# EUROPEAN PATENT SPECIFICATION

(11) **EP 2 050 453 B1**
(45) Date of publication and mention of the grant of the patent: **14.09.2011**
(21) Application number: 08170195.5
(22) Date of filing: 14.02.2003
(51) Int. Cl.: A61K 31/5415, A61K 31/485, A61K 31/167, A61P 25/00

(54) **Pharmaceutical combinations of meloxicam, tramadol and paracetamol**
Pharmazeutische Kombinationen bestehend aus Meloxicam, Tramadol und Paracetamol
Composes pharmaceutiques contenant du meloxicam, du tramadol et du paracetamol

(30) Priority: 19.02.2002 ZA 200201395
(43) Date of publication of application: 22.04.2009
(62) Divisional of application: 03706774.1
(73) Proprietor: ADCOCK INGRAM LIMITED, Bryanston 2021 (ZA)
(72) Inventor: Norris, Michael Christian, 2055, Fourways Gardens (ZA)
(74) Representative: Creek, Isobel Clare

(56) References cited:
- WO-A-97/20551
- WO-A-99/21585
- ZA-A- 8 405 785
- ZA-A- 8 903 422

## Description

### Background

This invention relates to pharmaceutical compositions for use in the symptomatic relief and treatment of pain, during algesic and/or hyperalgesic states, with or without fever.

A hyperalgesic state can be defined as the state of exaggerated response to painful stimuli. It is a behavioral state in which the threshold to potentially painful events is reduced, and reactions to supra-threshold painful events are exaggerated. Algesia describes a normal physiological response to pain.

Pain of any aetiology can be a debilitating problem. Numerous theories have been proposed on the cause and treatment of this pathological condition. A vast number of receptors, biochemical transmitters and physiological processes are involved in the sensation and response to painful stimuli. Most pharmacological modalities target one specific site to reduce painful symptoms, which frequently does not provide adequate pain relief.

### Summary of the Invention

According to one aspect of the invention, a pharmaceutical composition comprises a combination of meloxicam in a daily dose of 0.5 mg to 30 mg, tramadol in a daily dose of 25 mg to 500 mg and paracetamol in a daily dose of 60 mg to 4000 mg.

The invention extends to the use of a combination as defined above in the manufacture of a medicament for use in a method of providing symptomatic relief or treatment of pain, in an algesic and/or hyperalgesic state, with or without fever, in particular that associated with inflammation such as that associated with trauma, osteoarthritis, rheumatoid arthritis, non-inflammatory myalgia or dysmenorrhoea.

The invention also extends to the use of a combination as defined above in the manufacture of a medicament for use in the symptomatic relief or treatment of pain, in an algesic and/or hyperalgesic state, with or without fever.

### Brief Description of the Drawings

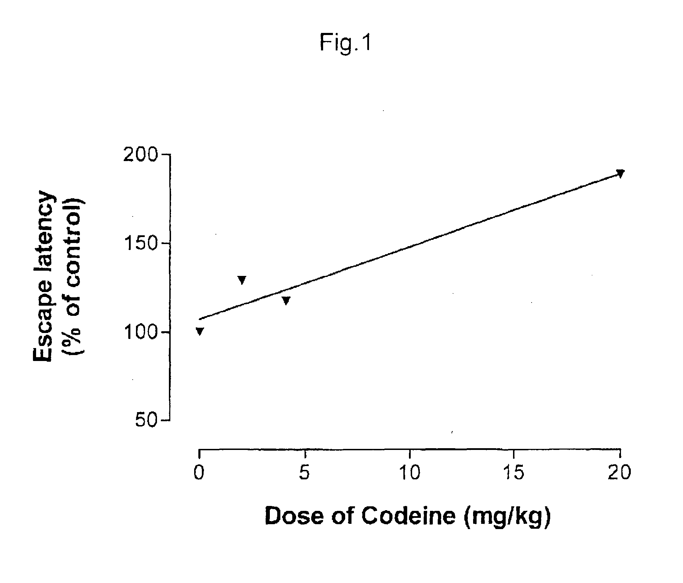

**Figure 1****:** A graphical representation of escape latency (median, n=10), expressed as a percentage of the escape latency in the same group of rats following administration of vehicle, following oral administration of meloxicam/codeine mixture in the ratio 1:4. Data is plotted against the codeine dose. ED₅₀ = 2.5 mg/kg meloxicam + 10.0 mg/kg codeine (r²=0.94, fit standard error=12%, log-linear regression analysis).

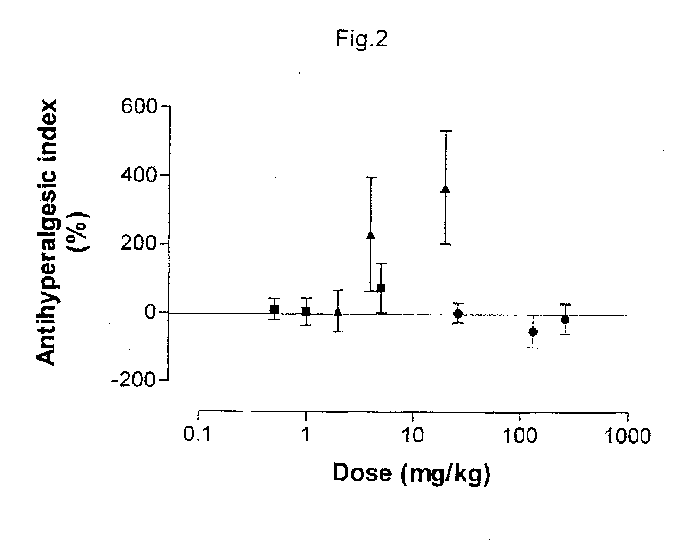

**Figure 2****:** A graphical representation of an antihyperalgesic index (mean ± SEM, n=9 or 10, different animals for each agent), calculated according to the formula of Sher *et al.* (1992), during reperfusion of the rat tail following transient ischaemia, after oral administration of meloxicam (squares), codeine (triangles) and paracetamol (circles) separately.

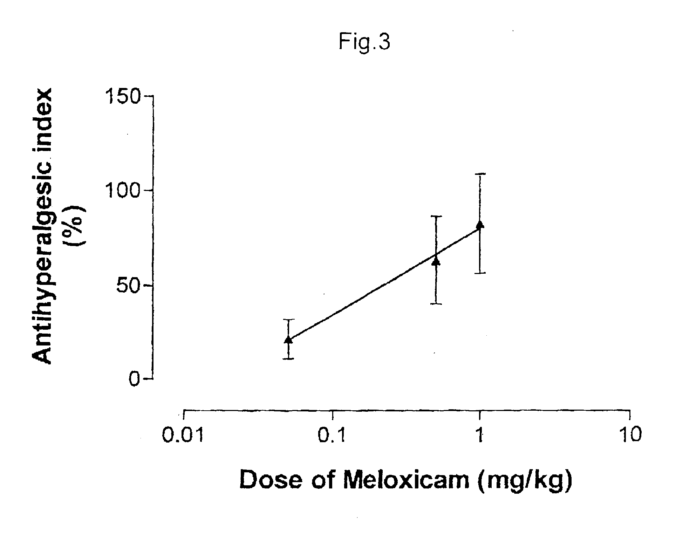

**Figure 3****:** A graphical representation of an antihyperalgesic index (mean ± SEM, n=9 or 10) during reperfusion of the rat tail following transient ischaemia, after oral administration of a mixture of meloxicam, codeine and paracetamol, in the ratio 1:4:266. Data is plotted against the meloxicam dose. ED₅₀ = 0.22 mg/kg meloxicam + 0.88 mg/kg codeine + 59 mg/kg paracetamol (r² = 0.99, fit standard error = 4%, log-linear regression analysis).

### Detailed Description of Preferred Embodiments

The pharmaceutical compositions of the invention are suitable for the symptomatic relief or treatment of pain, algesic and/or hyperalgesic, with or without fever, in particular but not limited to that associated with inflammatory processes, such as trauma, osteoarthritis, rheumatoid arthritis, non-inflammatory myalgia and dysmenorrhoea.

The first ingredient is meloxicam. This agent has anti-inflammatory and analgesic properties. This ability to inhibit the action of COX 2 and not COX 1 has been shown to provide an enhanced safety profile for this compound when compared to non-specific COX inhibitors.

The daily dose of the meloxicam active ingredient is in the range of 0.5 mg to 30 mg, preferably about 7.5 mg to about 15 mg.

The second ingredient is an opiate, tramadol. This compound binds with specific receptors at many sites within the central nervous system to alter processes affecting both the perception of pain and the emotional response to pain.

The daily dose of tramadol is in the range of 25mg to 500mg. An alternative opiate is codeine phosphate, although this does not form part of the invention.

A centrally-acting cyclo-oxygenase inhibitor is added to these compositions, which is the analgesic paracetamol (acetaminophen) or a pharmaceutically acceptable salt or derivative thereof. It has analgesic and antipyretic properties but limited or no anti-inflammatory action.

The daily dose of the paracetamol active ingredient is in the range of about 60 mg (children) to about 4000 mg (adults).

The pharmaceutical compositions of the invention include a pharmaceutically acceptable carrier and may include other necessary non-active excipients such as, for example, sorbitol, sucrose, saccharin, starch, lactose, guar gum, xanthan gum, magnesium stearate, bees wax, talc, methylcellulose, dextrin, povidone or polyvinylpyrrolidone. The pharmaceutical compositions may be provided in any appropriate dosage form such as, for example, tablets, capsules, granules, suspensions, solutions or other liquid forms, and are intended for oral, rectal, transdermal, intramuscular or intravenous administration.

The dosage form will typically be administered to a patient from 1 to 4 times per day. The product may be administered as an infusion, continuous or otherwise.

The invention will now be described, by way of example only, with reference to the following examples.

The challenge is to formulate a therapeutically rational dosage form based on the marked differences in the half-life and dosage quantities of the drugs in question. One possible means to circumvent these differences would be to incorporate the active ingredients into a mixed release granular dosage delivery system. It is proposed that the meloxicam would be immediately released from the formulation. The paracetamol and codeine/tramadol components would be released in appropriate quantities over a period of time.

Granules containing the paracetamol and codeine/tramadol components could be coated with pharmaceutically acceptable adjuvants such as sucrose, shellac, wax and guar gum. An example for the paracetamol component would be a 2 phase granular sustained release system:

| **Ingredient** | **Quantity** | **Purpose of Ingredient** |
|---|---|---|
| **Granule 1 (Sustained Release Phase 1)** | | |
| Paracetamol | 500 mg | Active |
| Sucrose | 825 mg | Diluent and Coating Agent |
| Maize Starch | 206 mg | Diluent |
| Shellac | 120 mg | Coating Agent |
| Talc | 200 mg | Lubricant |

| **Granule 2 (Sustained Release Phase 2)** | | |
|---|---|---|
| Paracetamol | 250 mg | Active |
| Sucrose | 550 mg | Diluent and Coating Agent |
| Maize Starch | 135 mg | Diluent |
| Shellac | 80 mg | Coating Agent |
| Talc | 130 mg | Lubricant |

In order to test the efficacy of a pharmaceutical composition containing codeine, the Brain Function Research Unit (BFRU) was engaged to investigate, in rats, the antinociceptive activity of a combination of meloxicam, codeine and paracetamol. That combination provides a unique consolidation of a COX 2-selective cyclo-oxygenase inhibitor, a weak opioid and the only currently-available COX 3 (i.e. overwhelmingly centrally-acting) inhibitor. Skill advancement in the Brain Function Research Unit also allowed the combination to be administered orally rather than intraperitoneally.

The assay used included a battery of tests developed by the BFRU over the last 17 years, and which has been shown to be useful in estimating the potencies of cyclo-oxygenase inhibitors marketed as non-steroidal anti-inflammatory drugs (NSAIDs) (Gelgor *et al.,* 1992). The scientific foundation of the battery recently has been validated independently, at a distinguished pain-research laboratory in Australia (Grace *et al.,* 2001). The battery includes a modified tail flick test, a test of sensitivity to noxious ischaemia, and a measurement of the hyperalgesia which develops during reperfusion to tissue following transient ischaemia (Gelgor *et al.,* 1986). The battery distinguishes analgesia, as produced, for example, by morphine (Sher *et al.,* 1992), from the antihyperalgesia produced by cyclo-oxygenase inhibitors (Mitchell 1999), as well as obviating ethical problems from which some of the other animal assays of antihyperalgesia suffer. It also identifies primary antinociceptive activity, rather than antinociceptive activity that might appear secondarily following resolution of inflammation. Because any test of antinociception in animals may produce false positive results if the antinociceptive agent under investigation compromises the animal's motor function (Cartmell *et al.,* 1991), it usually is necessary to check activity of any agents under test on motor function. However, previous research carried out by the BFRU has established that, at the doses under investigation, none of the three agents has any effect on motor function, so further checks on motor function were waived.

As there is no scientific evidence available upon which to constitute an appropriate ratio of meloxicam, codeine and paracetamol it was decided to employ the ratio of 1 meloxicam: 4 codeine: 266 paracetamol. That ratio was derived from the maximum daily doses of the three agents routinely employed clinically in South Africa, namely 15 mg meloxicam, 60 mg codeine and 4000 mg paracetamol. If antinociceptive activity could be demonstrated, it was believed that it may be possible to retreat from those high doses in follow-up investigations.

### Test Methods

### Animals

Sprague-Dawley rats (*Rattus norvegicus*) both genders weighing 250-450g were used for nociceptive tests. The rats were housed individually at an ambient temperature of 21-23°C on a 12-hour light; 12-hour dark cycle, and were allowed free access to standard rat chow and tap water. Groups of ten rats were used for each test. Rats were returned to stock, and in good health, after the tests.

### Tests of nociception

### Nociception during noxious ischaemia

Ischaemia was induced by applying an inflatable tourniquet to the base of the restrained rat's tail, as detailed previously (Gelgor *et al.,* 1992). The moment the rat exhibited an escape response, the tourniquet was deflated. The time between application of the tourniquet and the escape response, that is the escape latency, is a measure of sensitivity to the noxious stimulus induced by ischaemia. To prevent tissue damage, the tourniquet was removed if the rat had not responded within 30 min.

### Nociception during noxious thermal stimulation

The response of the rats to a noxious thermal stimulus was measured using a modified tail flick test, the details of which have previously been described (Gelgor *et al.,* 1992). The rat's tail was submerged in a water bath controlled at 49°C, and the time to the first coordinated motor response of the tail measured, with a safety cut-off at 30s. Tail skin temperature was maintained at 29°C by placing all but the proximal 20 mm of the rat's tail in a temperature-controlled bath, before and between measurements, to obviate the potential confounding effects of changes in tail temperature on tail flick latency (Tjolsen *et al.,* 1989).

### Hyperalgesia

The hyperalgesia which occurs during reperfusion of the tail following transient ischaemia, that is following the release of the tourniquet mentioned above, was measured by comparing the tail flick latency during reperfusion with that measured in the same animal before application of the tourniquet. Hyperalgesia is manifest as a reduced tail flick latency during reperfusion, and lasts about one hour (Gelgor *et al.,* 1992).

Control experiments were performed by placing a sham tourniquet on the tail for 20 min (mean escape latency measured in previous experiments in the laboratory), and administering the highest dose of each agent or combination.

### Procedure

Rats were habituated to restrainers for three hours per day on three consecutive days before any measurements were made. On the second of these days, the rats were given an oral dose of water, to allow them to experience the feeding tubes in advance. On experimental days, the animals were placed in restrainers for at least 30 min before any testing. At least 48 h were allowed between successive measurements on individual animals. Experiments were carried out during the day (when rats normally are quiescent), at an ambient temperature of 24°C.

The tail flick latency (mean of three measurements, 1 min apart) was measured and then the agent under test was administered. Thirty minutes later the tourniquet (or sham tourniquet) was applied and the escape latency measured. Immediately after release of the tourniquet, the tail flick latency was again measured, and the measurements were repeated after 30 and 60 min of reperfusion. Following inspection of the data at all three periods of reperfusion, the investigations elected to base their assessment of antihyperalgesic activity on data obtained immediately after release of the tourniquet.

### Agents and administration

Paracetamol, meloxicam and codeine phosphate were provided by the applicant. Codeine phosphate was dissolved in water. Meloxicam was dissolved in sunflower oil. Paracetamol was suspended in sunflower oil. All three agents were administered in boluses of 0.2 ml, and the paracetamol suspension was flushed with water. In control experiments, the appropriate vehicle (water, sunflower oil, or both) was administered.

Each agent was administered separately, at three doses, to determine the antinociceptive activity, if any, of each independently. Pairs of agents then were administered at three doses for each pair. Finally, the combination of the three agents was administered, at three doses. In all cases, the ratio of the agents was fixed at 1 meloxicam: 4 codeine: 266 paracetamol. The actual doses were based on unpublished data on the antipyretic activity of meloxicam administered orally to rats. The targeted maximum dose of meloxicam was 5 mg/kg (rats are much less sensitive to all cyclo-oxygenase inhibitors than are humans), for which the corresponding dose of paracetamol would have been 1333 mg/kg. It was not possible to make a suspension of paracetamol to administer such a high dose, so the targeted maximum doses of 5 mg/kg meloxicam and 20 mg/kg codeine were employed only when combination with paracetamol was not required.

Each group of ten rats received all the doses, and the appropriate control administration, for just one agent or combination of agents, which exposed each rat to five oral administrations after the pilot administration. The number of administrations in each rat was limited not as a result of fear of potential adverse events or toxicity, but to maintain the experimental animals in the same mass range throughout.

### Ethical considerations

The experimental procedures were approved by the Animal Ethics Committee of the University of the Witwatersrand (Certificate No. 2002/50/3) and complied with the recommendations of the Committee for Research and Ethical Issues of the International Association for the Study of Pain (Zimmerman, 1983).

### Results

### Analgesic (opioid-like) activity

Table 1 shows the escape latencies (the measure of sensitivity to the noxious ischaemic stimulus) following administration of the highest dose tested of each agent, or combination of agents, together with the escape latency for the same group of rats following administration of the appropriate vehicle. Because escape latencies vary between groups of rats, the efficacy of each active agent must be judged by comparing the two escape latencies within the same group of rats. Although escape latencies appeared to increase following administration of several of the agents and combinations, the only administration which produced a statistically significant increase in escape latency, that is statistically significant analgesia to noxious ischaemia, was a combination of meloxicam (5 mg/kg) and codeine (20 mg/kg).

**TABLE 1: Escape latency (median, 95% confidence interval, n=10) following application of noxious ischaemia to the tail, after oral administration 30 min earlier of each of the agents at the highest dose tested, and of vehicle in the same rats. Cut-off latency = 30 min.**

| **Agent** | **Dose (mg/kg)** | **Escape latency (min)** | |
|---|---|---|---|
| | | **Agent** | **Vehicle** |
| Meloxicam | 5 | 25.3 (15.7-30.0) | 19.3 (11.2-30.0) |
| Codeine | 20 | 20.9 (11.9-30.0) | 26.7 (11.7-30.0) |
| Paracetamol | 266 | 18.2 (9.2-30.0) | 17.2 (11.3-30.0) |
| Meloxicam | 5 | 25.1 (13.6-27.1)* | 13.3 (8.8-21.2) |
| + codeine | 20 | | |
| Meloxicam | 1 | 23.6 (10.9-30.0) | 15.0 (8.4-30.0) |
| + paracetamol | 266 | | |
| Paracetamol | 266 | 24.9 (9.1-30.0) | 16.7 (9.9-30.0) |
| + codeine | 4 | | |
| Meloxicam | 1 | 17.9 (10.8-27.0) | 16.4 (8.7-30.0) |
| + codeine | 4 | | |
| + paracetamol | 266 | | |

| | | | |
|---|---|---|---|
| * Significant increase, p=0.004, Friedman test | | | |

Figure 1 shows the relationship between the escape latency and dose for the meloxicam/codeine combination, and demonstrates that, at the highest dose, escape latency was increased by about 90%, which is evidence for substantial analgesic activity. If the combination of meloxicam and codeine produces analgesia, it would be expected that the triple combination formed by adding paracetamol would produce analgesia too. This could not be tested, however, because the dose of paracetamol corresponding to the 20 mg/kg dose of codeine was physically too large to administer. At the highest dose for the combination that could be tested, in which the codeine contribution was 4 mg/kg, there was no significant analgesia.

### Antihyperalgesic (COX-inhibitor-like) activity

Antihyperalgesia is measured by the extent to which an agent reverses the hyperalgesia induced by conditioning event, which, in the test case, was transient ischaemia. An antihyperalgesic index of zero means complete lack of antihyperalgesic activity, an index of 100% means full reversal of the hyperalgesia, and an index of more than 100% implies some analgesic activity added to the antihyperalgesic activity. Figure 2 shows the antihyperalgesic index, at three doses of each of the agents, administered separately. At the doses tested, which were limited to 266 mg/kg, paracetamol did not exhibit any antihyperalgesic activity. Even though their doses were much lower, both meloxicam and codeine did exhibit dose-dependent antihyperalgesia activity, even when administered separately.

Figure 3 shows the antihyperalgesic index exhibited when the triple combination of meloxicam, codeine and paracetamol was administered. Dose-dependent antihyperalgesia was evident, even though the doses of the individual agents in the combination were much lower than those required to produce antihyperalgesia separately. An ED₅₀ value was calculated from regression analysis applied to the data, and is shown, together with the ED₅₀ values for the individual agents, in Table 2.

**TABLE 2: ED₅₀ (dose at which reperfusion hyperalgesia is reduced by 50%) for the antihyperalgesic efficacy of the combination of meloxicam, codeine and paracetamol, and for each agent separately. From least-squares regression lines fitted to the dose-response curves, n=9 or 10 for each test.**

| **Agent** | **ED₅₀ (mg/kg)** |
|---|---|
| Meloxicam | 0.22 |
| + codeine | 0.88 |
| + paracetamol | 59 |
| Meloxicam | 2.6 |
| Codeine | 2.4 |
| Paracetamol | >266 |

The ED₅₀ value for the triple combination was 0.22 mg/kg meloxicam plus 0.88 mg/kg codeine plus 59 mg/kg paracetamol. Inspection of Figure 2 shows, and regression analysis confirmed, that at a dose of 0.22 mg/kg, meloxicam on its own showed no antihyperalgesic activity. Similarly, neither did codeine at 0.88 mg/kg nor paracetamol at 59 mg/kg. Thus, in triple combination, the three components were antihyperalgesic at doses at which the components individually were inactive, which is an indication of their synergistic activity when in combination.

The antihyperalgesic activity of the individual components was also tested in pairs, rather than as a triple combination. At the doses tested, paracetamol and codeine together had no significant antihyperalgesic activity. Meloxicam and codeine indeed did have significant antihyperalgesic activity, with an ED₅₀ of 1.7 mg/kg meloxicam and 6.8 mg/kg codeine, that is about ten times higher than the ED₅₀ doses in triple combination. Meloxicam and paracetamol also had antihyperalgesic activity, and with an ED₅₀ not significantly different from that of the triple combination, but the investigators did not have confidence in the ED₅₀ value calculated for the meloxicam/paracetamol pair, because of high inter-individual variability in the rats in that particular cohort.

### Adverse events

Formal tests for adverse events were not part of the study design. However, the animals remained alert, active and in apparent good health throughout the study. Also, all animals continued to gain weight throughout the study; loss of weight is an early sign of pathology in rats. The animals were housed in the Central Animal Service of the University under veterinary care, and no adverse events were recorded on the veterinary bedletters. As far as the active ingredients tested are concerned, no interactions between the actives of an adverse nature were observed.

### Conclusions

From the above it was demonstrated that a triple combination of meloxicam and codeine and paracetamol, administered orally in a single dose to rats, produces significant antihyperalgesic activity, that is ability to relieve pain of the type which occurs in indications in which the cyclo-oxygenase inhibitors (NSAIDs, COXIBs, non-narcotic analgesics) are functional. The dose of meloxicam required, when in combination with codeine and paracetamol, is about one tenth of the dose of meloxicam required if it is administered as a single agent, which means that combining meloxicam with codeine and paracetamol enhances the antihyperalgesic efficacy of meloxicam by about tenfold. This enhancement results from synergies between the three components, because the ED₅₀ of the triple combination is composed of doses at which each of the three components is inactive on its own. Thus the triple combination of meloxicam and codeine and paracetamol is a candidate synergistic antihyperalgesic product.

Although no formal assessments of adverse events associated with the administration of the triple combination was made, each rat in the relevant cohort received the triple combination on four occasions in various doses, which included twice receiving a dose five times higher than the ED₅₀. The investigators did not observe a change in the weight gain, demeanor, activity or vital signs of the rats, and they were in excellent condition at the end of the study. They had no reason to expect synergy of adverse events in the triple combination.

The above also demonstrates that a combination of meloxicam and codeine produces significant analgesic activity, that is ability to relieve pain of the type which occurs in indication in which the opioids are active. The ED₅₀ dose of meloxicam and codeine necessary to produce significant analgesic activity was about ten times higher than their contributions in the ED₅₀ dose of the triple combination for antihyperalgesic activity. Physical constraints related to the insolubility of paracetamol prevented the investigation of whether addition of paracetamol, in the appropriate ratio, to the meloxicam/codeine combination would enhance the analgesic activity. However, it is believed to be highly unlikely that the paracetamol would reduce analgesic activity, so, by extrapolation, the triple combination of meloxicam, codeine and paracetamol will produce significant opioid-like analgesic activity, but only at doses much higher than the doses necessary for antihyperalgesic activity.

As the triple combination provides both antihyperalgesic (cyclo-oxygenase inhibitor-like) and analgesic (opioid-like) activity, it is a candidate for a medication indicated for a wide range of types of pain, both conditioned pain (e.g. pain and inflammation, dysmenorrhoea) and unconditioned pain (e.g. non-inflammatory myalgia).

The combination of meloxicam and codeine, without any paracetamol, produced significant opioid-like analgesia, but needed a high dose of codeine. Tramadol is another opiate which has greater analgesic efficacy than codeine, and does not carry with it the perceptions of causing constipation, and abuse potential, which codeine has. It therefore seems reasonable that a combination of meloxicam with tramadol would produce better analgesia, and consequently allow either lower doses, or positioning at greater potency, than a meloxicam/codeine mixture which was tested.

The synergistic effect of the active ingredient is believed to provide a pharmaceutical product which delivers high antihyperalgesic effects while each active may be at a sub-therapeutic dose. This, it is believed, will provide a product with a low side effect profile. Furthermore due to the low doses of actives the pharmaceutical product cost for treating hyperalgesic states could be reduced.

### References

CARTMELL, S.M., GELGOR, L. & MITCHELL, D. (1991). A revised rotarod procedure for measuring the effect of antinociceptive drugs on motor function in the rat. J. Pharmacol. Methods, 26, 140-159.
GELGOR, L., PHILLIPS, S. & MITCHELL, D. (1986). Hyperalgesia following ischaemia of the rat's tail. Pain, 24, 251-257.
GELGOR, L., BUTKOW, N. & MITCHELL, D. (1992). Effects of systemic non-steroidal anti-inflammatory drugs on nociception during tail ischaemia and on reperfusion hyperalgesia in rats. Br. J. Pharmacol., 105, 412-416.
GRACE, R.F., LYN, Y., EDWARDS, S.R., POWER, I. & MATHER, L.E. (2001). Effects of diclofenac in the rat tail ischaemia-reperfusion model of acute hyperalgesia. Pain, 89, 117-125.
MITCHELL, D. (1999). Hyperalgesia. Specialist Med., 21, 463-469.
SHER, G.D., CARTMELL, S.M., GELGOR, L. & MITCHELL, D. (1992). Role of N-methyl-D-aspartate and opiate receptors in nociception during and after ischaemia in rats. Pain, 49, 241-248.
TJOLSEN, A., LUND, A., BERGE, O-G. & HOLE, K. (1989). An improved method for tail-flick testing with adjustment for tail-skin temperature. J. Neurosci. Meth., 26, 259-265.
ZIMMERMAN, M. (1983). Ethical guidelines for investigations of experimental pain in conscious animals. Pain, 16, 109-110.

## Claims

1. A pharmaceutical composition comprising a combination of meloxicam in a daily dose of 0.5 mg to 30 mg, tramadol in a daily dose of 25 mg to 500 mg and paracetamol in a daily dose of 60 mg to 4 000 mg.

2. A pharmaceutical composition according to claim 1, wherein the meloxicam, tramadol and paracetamol are provided in amounts such that the combination is therapeutically effective in treating pain, in an algesic and/or hyperalgesic state, with or without fever.

3. A combination of meloxicam in a daily dose of 0.5 mg to 30 mg, tramadol in a daily dose of 25 mg to 500 mg and paracetamol in a daily dose of 60 mg to 4 000 mg as active ingredients, for use in the treatment of pain, in an algesic and/or hyperalgesic state, with or without fever, the amounts of the active ingredients being selected such that the combination is therapeutically effective.

4. The combination of claim 3, wherein the pain is associated with inflammation.

5. The combination according to claim 4, wherein the pain is associated with trauma, osteoarthritis, rheumatoid arthritis, non-inflammatory myalgia or dysmenorrhoea.

6. The use of a combination of meloxicam in a daily dose of 0.5 mg to 30 mg, tramadol in a daily dose of 25 mg to 500 mg and paracetamol in a daily dose of 60 mg to 4 000 mg, as active ingredients, in the manufacture of a medicament for use in treating pain, in an algesic and/or hyperalgesic state, with or without fever.

7. The use of claim 6, wherein the medicament is provided in a dosage form selected from the group comprising tablets, capsules, granules, suspensions, solutions and other liquid forms.

8. The use of claim 7, wherein the medicament is for administration orally, rectally, transdermally, intramuscularly or intravenously.

9. The use of claim 8, wherein the dosage form is for administration to a patient from 1 to 4 times a day.

10. The use of claim 7, wherein the medicament is for administration as an infusion, continuous or otherwise.

## Patentansprüche

1. Pharmazeutische Zusammensetzung, die eine Kombination aus Meloxicam in einer Tagesdosis von 0,5 mg bis 30 mg, Tramadol in einer Tagesdosis von 25 mg bis 500 mg und Paracetamol in einer Tagesdosis von 60 mg bis 4.000 mg umfasst.

2. Pharmazeutische Zusammensetzung nach Anspruch 1, wobei das Meloxicam, das Tramadol und das Paracetamol in solchen Mengen bereitgestellt werden, dass die Kombination bei der Behandlung von Schmerz, bei Zuständen der Schmerzempfindlichkeit und/oder der übermäßigen Schmerzempfindlichkeit, mit oder ohne Fieber, therapeutisch wirksam ist.

3. Kombination aus Meloxicam in einer Tagesdosis von 0,5 mg bis 30 mg, Tramadol in einer Tagesdosis von 25 mg bis 500 mg und Paracetamol in einer Tagesdosis von 60 mg bis 4.000 mg als wirksame Inhaltsstoffe zur Verwendung bei der Behandlung von Schmerz, bei Zuständen der Schmerzempfindlichkeit und/oder der übermäßigen Schmerzempfindlichkeit, mit oder ohne Fieber, wobei die Mengen der wirksamen Inhaltsstoffe derart ausgewählt werden, dass die Kombination therapeutisch wirksam ist.

4. Kombination nach Anspruch 3, wobei der Schmerz mit einer Entzündung verbunden ist.

5. Kombination nach Anspruch 4, wobei der Schmerz mit einem Trauma, Arthrose, Gelenkrheumatismus, nicht entzündlicher Myalgie oder Dysmenorrhoe verbunden ist.

6. Verwendung einer Kombination von Meloxicam in einer Tagesdosis von 0,5 mg bis 30 mg, Tramadol in einer Tagesdosis von 25 mg bis 500 mg und Paracetamol in einer Tagesdosis von 60 mg bis 4.000 mg als wirksame Inhaltsstoffe bei der Herstellung eines Medikaments zur Verwendung bei der Behandlung von Schmerz, bei Zuständen der Schmerzempfindlichkeit und/oder der übermäßigen Schmerzempfindlichkeit, mit oder ohne Fieber.

7. Verwendung nach Anspruch 6, wobei das Medikament in einer Darreichungsform bereitgestellt wird, die aus der Gruppe, die Tabletten, Kapseln, Granulat, Suspensionen, Lösungen und anderen flüssigen Formen umfasst, ausgewählt ist.

8. Verwendung nach Anspruch 7, wobei das Medikament zur oralen, rektalen, transdermalen, intramuskulären oder intravenösen Verabreichung vorgesehen ist.

9. Verwendung nach Anspruch 8, wobei die Darreichungsform zur Verabreichung an einen Patienten von 1- bis 4-mal am Tag vorgesehen ist.

10. Verwendung nach Anspruch 7, wobei das Medikament zur Verabreichung als eine kontinuierliche oder andere Infusion vorgesehen

## Revendications

1. Composition pharmaceutique comprenant une combinaison de meloxicam dans une dose journalière de 0,5 mg à 30 mg, de tramadoldans une dose journalière de 25 mg à 500 mg et de paracétamol dans une dose journalière de 60 mg à 4000 mg.

2. Composition pharmaceutique selon la revendication 1, dans laquelle le meloxicam, le tramadolet le paracétamol sont fournis dans des quantités telles que la combinaison est thérapeutiquement efficace pour traiter la douleur, un état algique et/ou hyperalgique, avec ou sans fièvre.

3. Combinaison de meloxicam, dans une dose journalière de 0,5 mg à 30 mg, de tramadoldans une dose journalière de 25 mg à 500 mg et de paracétamol dans une dose journalière de 60 mg à 4000 mg, comme ingrédients actifs, à utiliser dans le traitement de la douleur, dans un état algique et/ou hyperalgique, avec ou sans fièvre, les quantités d'ingrédients actifs étant choisies de sorte que la combinaison soit thérapeutiquement efficace.

4. Combinaison selon la revendication 3, dans laquelle la douleur est associée à une inflammation.

5. Combinaison selon la revendication 4, dans laquelle la douleur est associée à un traumatisme, à de l'arthrose, à une polyarthrite rhumatoïde, à une myalgie non inflammatoire ou à une dysménorrhée.

6. Utilisation d'une combinaison de meloxicam dans une dose journalière de 0,5 mg à 30 mg, de tramadoldans une dose journalière de 25 mg à 500 mg et de paracétamol dans une dose journalière de 60 mg à 4000 mg, comme ingrédients actifs, dans la fabrication d'un médicament destiné à traiter la douleur, un état algique et/ou hyperalgique, avec ou sans fièvre.

7. Utilisation selon la revendication 6, dans laquelle le médicament est fourni sous une forme galénique choisie dans le groupe comprenant les comprimés, les gélules, les granules, les suspensions, les solutions et autres formes liquides.

8. Utilisation selon la revendication 7, dans laquelle le médicament est destiné à une administration par voie orale, rectale, transdermique, intramusculaire ou intraveineuse.

9. Utilisation selon la revendication 8, dans laquelle la forme galénique est pour administration à un patient entre 1 et 4 fois par jour.

10. Utilisation selon la revendication 7, dans laquelle le médicament est destiné à être administré par perfusion, continue ou autre.
